(19) Europäisches Patentamt
European Patent Office
Office européen des brevets

(11) Veröffentlichungsnummer: **0 029 123**
**B1**

# (12) EUROPÄISCHE PATENTSCHRIFT

(45) Veröffentlichungstag der Patentschrift: **08.06.83**

(21) Anmeldenummer: **80106324.9**

(22) Anmeldetag: **17.10.80**

(51) Int. Cl.³: **C 07 C 93/14,** C 07 C 121/75, C 07 C 149/42, C 07 D 213/65, C 07 D 237/14

(54) **Verfahren zur Herstellung von substituierten Anilinen und neue substituierte Aniline.**

(30) Priorität: **24.10.79 DE 2942930**
**05.04.80 DE 3013267**
**09.09.80 DE 3033836**

(43) Veröffentlichungstag der Anmeldung:
**27.05.81 Patentblatt 81/21**

(45) Bekanntmachung des Hinweises auf die Patenterteilung:
**08.06.83 Patentblatt 83/23**

(84) Benannte Vertragsstaaten:
**AT BE CH DE FR GB IT LI NL SE**

(56) Entgegenhaltungen:
**DE-A-1 593 871**
**DE-A-2 062 349**
**DE-A-2 311 638**
**DE-A-2 333 848**
**DE-A-2 504 982**

**„Chemical Abstracts" Bd. 92, Juni 1980, Columbus, Ohio, USA, R. Nishiyama *et al.* „N-Benzoyl-N'-pyridyloxyphenylureas", S. 639, Spalte 1, „Abstract" Nr. 181018e in Verbindung mit „Formula Index" Bd. 92, S. 717F, Spalte 2, Zeile 74.**

(73) Patentinhaber: **BASF Aktiengesellschaft, Carl-Bosch-Strasse 38, D-6700 Ludwigshafen (DE)**

(72) Erfinder: **Parg, Adolf, Dr., Paray-le-Monial-Strasse 8, D-6702 Bad Duerkheim (DE)**
Erfinder: **Hamprecht, Gerhard, Dr., Rote-Turm-Strasse 28, D-6940 Weinheim (DE)**
Erfinder: **Oeser, Heinz-Guenter, Dr., Mahngasse 25, D-6711 Dirmstein (DE)**

**EP 0 029 123 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von substituierten Anilinen und neue substituierte Aniline

Die vorliegende Erfindung betrifft ein Verfahren zur Herstellung von substituierten Anilinen und neue substituierte Aniline.

In der Fachliteratur wird als gebräuchliche Methode zur Herstellung von phenoxy- bzw. heteroaryloxysubstituierten Anilinen die Reduktion der entsprechenden Nitroverbindungen angegeben (Houben-Weyl, „Methoden der Organischen Chemie", Bd. 11/1, S. 342 bis 600). Der Nachteil dieser Verfahrensweise besteht hauptsächlich in dem Einsatz eines meist teueren Reduktionsmittels und eines für Nitroverbindungen allgemein notwendigen sicherheitstechnischen Aufwandes. Zudem sind viele phenoxy- bzw. heteroaryloxysubstituierte Nitrobenzole, insbesondere die metasubstituierten Derivate, nur schwer zugänglich.

Aus der GB-PS Nr. 1064115 ist bekannt, dass man aus dem Alkalisalz von Phenol mit 3-Chloranilin unter Zusatz von Kupferbronze in der Schmelze bei 220 bis 250° C das entsprechende Phenoxyanilin in mässigen Ausbeuten erhält. Diese Methode erfordert einen hohen Energieeinsatz und versagt bei temperaturempfindlichen Einsatzstoffen, so dass ihre Anwendungsbreite sehr begrenzt ist.

Ebenso ist aus den DE-OS Nrn. 1593871 und 2062349 sowie aus „Liebigs Annalen der Chemie", Bd. 740, S. 169 bis 179 (1970) bekannt, dass man Alkaliaminophenolate mit besonders reaktiven aromatischen Halogenverbindungen, in Gestalt von ausschliesslich Nitrochlorbenzolen oder Chlorbenzonitrilen, an der Phenolgruppe umsetzen kann, wobei Aminophenoxynitrobenzole bzw. Aminophenoxybenzonitrile entstehen. Diese Reaktionen ergeben jedoch die genannten Verbindungen nur dann in guten Ausbeuten, wenn man entweder Dimethylsulfoxid oder Dimethylformamid mit äquivalenten Mengen Kaliumcarbonat verwendet und mässig erhöhte Temperaturen einhält. Bei zu hohen Temperaturen oder ungeeigneter Reaktionsführung tritt nämlich auch die reaktionsfähige Aminogruppe unter Bildung von Diphenylaminderivaten in die Reaktion ein. Darüber hinaus sind die Aminophenole, besonders im basischen Milieu, bekanntermassen oxydationsempfindliche Substanzen, die leicht in gefärbte Zersetzungsprodukte übergehen.

Es ist aus DE-OS Nr. 2333848 bekannt, dass man 4-Halogenbenzotrifluoride mit Phenolen oder Thiophenolaten zu halogenierten 4-Trifluormethyldiphenyläthern oder -thioäthern umsetzt.

Ebenfalls wird (S. 32) die Herstellung von 2-Chlor-4'-amino-4-trifluormethyldiphenyläther aus Natrium-4-aminophenolat und 3,4-Dichlorbenzotrifluorid in Dimethylsulfoxid bei 120-140° C beschrieben. Die Angabe einer Ausbeute fehlt. Wie aber Vergleichsbeispiel 1a im Vergleich zu dem erfindungsgemässen Beispiel 1 zeigt, erhält man in Abwesenheit von Kronenäthern als Katalysator nur eine Ausbeute von 14%. Demgegenüber steigert sich die Ausbeute bei Zusatz von Kronenäthern als Katalysator auf insgesamt 78% (Beispiel 1).

Alle vorgenannten Verfahren sind in bezug auf Ausbeute, Reaktionsdauer, Reinheit und Vielzahl der Endstoffe, einfachen, wirtschaftlichen Betrieb, Energiebedarf und Umweltfreundlichkeit, gerade auch im grosstechnischen Massstab, unbefriedigend.

Es wurde nun gefunden, dass man substituierte Aniline der Formel

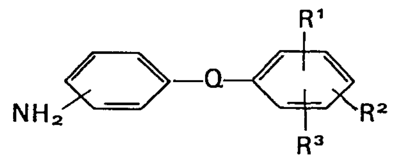

(I)

worin in dem aminogruppenfreien aromatischen Ring 1 oder 2 CH-Gruppen durch Stickstoffatome ersetzt sein können, die Reste $R^1$, $R^2$ und $R^3$ gleich oder verschieden sein können und Wasserstoffatome, Halogenatome, Nitrogruppen, Cyangruppen, Trihalogenmethylgruppen, $C_{1\ bis\ 4}$-Alkoxyreste, $C_{1\ bis\ 4}$-Alkylmercaptoreste, aliphatische Reste mit 1 bis 4 Kohlenstoffatomen, aliphatische Sulfonylreste mit 1 bis 4 Kohlenstoffatomen, aliphatische Sulfoxidreste mit 1 bis 4 Kohlenstoffatomen oder Carbalkoxyreste mit 2 bis 5 Kohlenstoffatomen und Q ein Sauerstoff- oder Schwefelatom bezeichnen durch Umsetzung eines Phenolsalzes mit aromatischen oder heterocyclischen Halogenverbindungen vorteilhaft erhält, wenn man Verbindungen der Formel

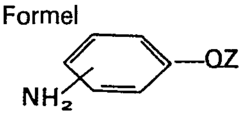

(II)

worin Q die vorgenannte Bedeutung besitzt und Z ein Metallkation bedeutet, mit Halogenaromaten oder Halogenheterocyclen der Formel

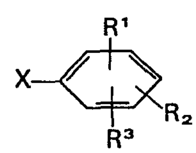

(III)

worin X ein Halogenatom bedeutet, $R^1$, $R^2$ und $R^3$ die vorgenannte Bedeutung besitzen und 1 oder 2 CH-Gruppen im Ring durch Stickstoffatome ersetzt sein können, in Gegenwart eines Kronenäthers als Katalysator und polarer aprotischer Lösungsmittel bei einer Temperatur zwischen 80 und 280° C umsetzt.

Weiterhin betrifft die Erfindung die neuen 2'-Chlor-4'-trifluormethyl-3-aminodiphenyläther, 3'-Chlor-4'-trifluormethyl-3-aminodiphenyläther, 2'-Nitro-4'-trifluormethyl-3-aminodiphenyläther, 3,2'-Brom-5'-pyridinoxyanilin und 2'-Chlor-4'-trifluormethyl-3-aminodiphenylthioäther.

Die Umsetzung kann im Falle der Verwendung von Natrium-3-aminophenolat und 3,4-Dichlortirfluormethylbenzol durch die folgenden Formeln ausgedrückt werden:

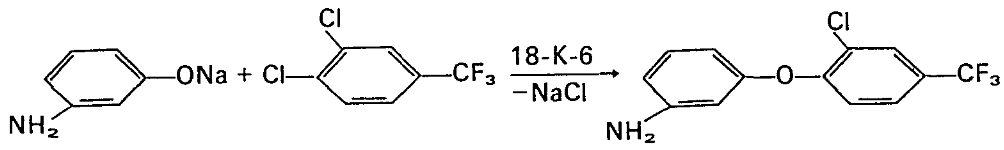


Im Hinblick auf die bekannten Verfahren liefert das Verfahren nach der Erfindung auf einfacherem und wirtschaftlicherem Wege eine bessere Ausbeute und Reinheit des Endstoffes. Schwer zugängliche Reaktionskomponenten wurden vermieden. Weitere Vorteile des neuen Verfahrens sind kurze Reaktionsdauer und hohe Reinheit, da praktisch kaum Anfall an Diphenylaminen eintritt, selektive Reaktion nur an der Phenolgruppe, leichte Herstellbarkeit auch von Metaaminodiphenyläthern, grosse Variationsbreite der Reaktion betreffend Lösungsmittel, Reaktionszeit und Temperatur. Ebenso war nicht vorhersehbar, dass die Umsetzung keine Nitrogruppen und/oder Cyangruppen zur Aktivierung der Umsetzung benötigt. Alle diese erfindungsgemässen Vorteile sind im Hinblick auf den Stand der Technik überraschend.

Die Ausgangsstoffe können in stöchiometrischer Menge oder im Überschuss, zweckmässig in einer Menge von 1 bis 2 mol, insbesondere 1,2 bis 1,5 mol Ausgangsstoff III je Mol Ausgangsstoff II, umgesetzt werden. Bevorzugte Ausgangsstoffe III und dementsprechend bevorzugte Endstoffe I sind solche, die nur Kohlenstoffatome im Molekülring tragen oder Kohlenstoffringe, die 1 oder 2 Stickstoffatome anstelle von CH-Gruppen tragen, und in denen die Reste $R^1$, $R^2$ und $R^3$ gleich oder verschieden sein können und jeweils Wasserstoffatome, Bromatome oder vorteilhaft Chloratome, Nitrogruppen, Cyangruppen, Trifluormethylgruppen, $C_{1\ bis\ 4}$-Alkoxyreste, $C_{1\ bis\ 4}$-Alkylmercaptoreste, Alkylreste mit jeweils 1 bis 4 Kohlenstoffatomen, einen aliphatischen Alkylsulfonylrest mit 1 bis 4 Kohlenstoffatomen, einen Alkylsulfinylrest mit 1 bis 4 Kohlenstoffatomen, einen Carbalkoxyrest mit 2 bis 5 Kohlenstoffatomen bedeuten. Z ist zweckmässig ein Metall der Gruppe Ia, IIa, IIb, IIIa, IIIb, IVb, Vb, VIb, VIIb und VIIIb des periodischen Systems, bevorzugt Alkali, insbesondere Natrium und Kalium, X ist ein Brom- oder zweckmässiger ein Chloratom.

Als Ausgangsstoffe II kommen Aminophenole und Aminothiophenole und als Ausgangsstoffe III aromatische Halogenverbindungen, halogensubstituierte Pyridine, Pyridazine, Pyrimidine und Pyrazine in Betracht. Geeignet sind z.B. folgende Aminophenole in Gestalt ihrer Salze als Ausgangsstoff II: das Natrium- oder Kaliumsalz von Aminophenol-(1) bzw. Aminothiophenol-(1) (mit dem Aminorest vorzugsweise in der 3-Stellung) sowie entsprechende Zink-, Barium-, Calcium-, Magnesium- oder Strontiumsalze.

Als Halogenaromaten bzw. halogenierte Heteroaromaten als Ausgangsstoff III eignen sich Verbindungen, die in 2-, 3-, 4-, 5- oder 6-Stellung am aromatischen oder heterocyclischen Ring durch Methyl-, Äthyl-, Propyl-, Isopropyl-, Butyl-, Isobutyl-, sek.-Butyl-, tert.-Butyl-, Methoxy-, Äthoxy-, Propoxy-, Isopropoxy-, Butoxy-, Isobutoxy-, sek.-Butoxy-, tert.-Butoxy-, Methylmercapto-, Äthylmercapto-, Propylmercapto-, Isopropylmercapto-, Butylmercapto-, Isobutylmercapto-, sek.-Butylmercapto, tert.-Butylmercaptogruppen, Brom, Jod, Nitro-, Cyan-, Trifluormethylgruppen; durch Methylsulfonyl-, Methylsulfinyl- oder Carbalkoxygruppe mit jeweils 2 bis 5 Kohlenstoffatomen je Alkylgruppe ein- bis dreifach substituiert sind. Bevorzugt sind als Halogenaromaten III: 4-Chlortrifluormethylbenzol, 2-Chlortrifluormethylbenzol, 3,4-Dichlortrifluormethylbenzol, 2,4-Dichlortrifluormethylbenzol, 3,5-Dichlortrifluormethylbenzol, 2,3-Dichlortrifluormethylbenzol, 4-Chlornitrobenzol, 3,4-Dichlornitrobenzol, 4-Chlorbenzonitril, 3,4-Dichlorbenzonitril, 3-Nitro-4-Chlortrifluormethylbenzol, 3-Nitro-2,4-dichlortrifluormethylbenzol, 3,4-Dichlormonofluormethylbenzol. Als Heteroaromaten III sind besonders bevorzugt: 3,6-Dichlorpyridazin, 3-Trifluormethyl-6-chlorpyridazin, 2,5-Dichlorpyridin, 2,5-Dibrompyridin, 2-Chlor-5-nitropyridin, 2-Chlor-5-cyanpyridin, 2-Chlor-5-trifluormethylpyridin, 2,3-Dichlor-5-trifluormethylpyridin, 2,3,5-Trichlorpyridin, 2,3,5-Tribrompyridin, 2,3-Dichlor-5-nitropyridin.

Die Umsetzung wird bei einer Temperatur zwischen 80 bis 280° C, vorzugsweise von 80 bis 160° C, insbesondere von 85 bis 140° C, drucklos oder unter Druck, kontinuierlich oder diskontinuierlich durchgeführt. Man verwendet unter den Reaktionsbedingungen inerte polare und aprotische Lösungsmittel. Als Lösungsmittel kommen z.B. in Frage: aromatische Kohlenwasserstoffe, z.B. Toluol, Äthylbenzol, o-, m-, p-Xylol, Isopropylbenzol, Methylnaphthalin; Sulfoxide wie Dimethylsulfoxid, Diäthylsulfoxid, Dimethylsulfon, Diäthylsulfon, Methyläthylsulfon, Tetramethylensulfon; aliphatische oder cycloaliphatische Kohlenwasserstoffe, z.B. α-Pinen, Pinan, Nonan, Benzinfraktionen innerhalb eines Siedepunktintervalls von 80 bis 190° C, Cyclohexan, Methylcyclohexan, Dekalin, Ligroin, 2,2,4-Trimethylpentan, 2,2,3-Trimethylpentan, 2,3,3-Trimethylpentan, Octan; Dimethylformamid; und entsprechende Gemische. Bevorzugte Lösungsmittel sind z.B. Dimethylformamid, Dimethylacetamid, N-Methylcaprolactam, N-Methylpyrrolidon, Tetramethylenharnstoff, Dimethylsulfoxid, Diäthylsulfoxid, Dipropylsulfoxid, Tetramethylensulfon, Hexametyhlenphosphorsäuretriamid, Acetonitril. Zweckmässig verwendet man das Lösungsmittel in einer Menge von 100 bis 2000 Gewichtsprozent, vorzugsweise von 250 bis 800 Gewichtsprozent, bezogen auf Ausgangsstoff II.

Bevorzugte Lösungsmittel sind Dimethylformamid, insbesondere aliphatische Sulfone und Sulfoxide, vorteilhaft der Formel

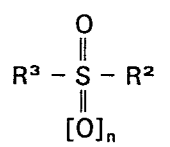 (IV)

worin $R^3$ und $R^2$ gleich oder verschieden sind und jeweils einen aliphatischen Rest, vorzugsweise einen Alkylrest mit 1 bis 8, insbesondere 1 bis 4 Kohlenstoffatomen oder $R^3$ und $R^2$ zusamment einen Alkylenrest mit 4 oder 5 Kohlenstoffatomen bedeuten und n 0 oder 1 bezeichnet. Geeignete Lösungsmittel IV sind z.B. Dimethylsulfoxid, Diäthylsulfoxid, Dipropylsulfoxid, Diisopropylsulfoxid, Di-n-butylsulfoxid, Diisobutylsulfoxid, Dipentylsulfoxid, Dihexylsulfoxid, Diheptylsulfoxid, Dioctylsulfoxid, Methyläthylsulfoxid, Tetramethylensulfoxid, Pentamethylensulfoxid, Dimethylsulfon, Diäthylsulfon, Dipropylsulfon, Diisopropylsulfon, Dibutylsulfon, Diisobutylsulfon, Dipentylsulfon, Dihexylsulfon, Diheptylsulfon, Dioctylsulfon, Methyläthylsulfon, Tetramethylensulfon, Pentamethylensulfon; besonders bevorzugt ist Tetramethylensulfon. Auch Lösungsmittelgemische können verwendet werden. Zweckmässig verwendet man die Lösungsmittel IV in einer Menge von 100 bis 2000 Gewichtsprozent, vorzugsweise von 250 bis 800 Gewichtsprozent, bezogen auf Ausgangsstoff II.

Als Katalysatoren verwendet man Kronenäther. Man setzt den Katalysator in stöchiometrischer Menge oder im Unterschuss ein, zweckmässig in einer Menge von 0,05 bis 0,3, insbesondere von 0,1 bis 0,15 mol.% Katalysator je Mol Ausgangsstoff II. Kronenäther sind organische Komplexliganden, die insbesondere gut zur Bindung von Alkali dienen, und cyclisch angeordnete, neutrale Äthylenglykoläther. Bezüglich Herstellung, Eigenschaften und Verwendbarkeit verweisen wir auf „Kontakte" (1977), S. 11 bis 31 und 36 bis 48.

Z.B. sind folgende Stoffe zweckmässig: 12-Krone-4; 2,4,6,8-Methyl-12-krone-4; 14-Krone-4; Dibenzo-14-krone-4; Dibutylbenzo-14-krone-4; Dicyclohexyl-14-krone-4; 15-Krone-5; 1,2-Benzo-15-krone-5; 1,2-Butylbenzo-15-krone-5; 1,2-Cyclohexyl-15-krone-5; Dibenzo-15-krone-5; 16-Krone-5; Dibenzo-16-krone-5; 18-Krone-5; Dibenzo-18-krone-5; 18-Krone-6; Benzo-18-krone-6; Cyclohexyl-18-krone-6; Dibenzo-18-krone-6; Dicyclohexyl-18-krone-6; Tribenzo-18-krone-6; Dinaphtho-18-krone-6; 19-Krone-6; Dibenzo-19-krone-6; 20-Krone-7; Dibenzo-5-oxy-20-krone-7; 21-Krone-7; Dibenzo-21-krone-7; Dicyclohexyl-21-krone-7; 24-Krone-8; Dibenzo-24-krone-8; Dicyclohexyl-24-krone-8; Tetrabenzo-24-krone-8; 30-Krone-10; 40-Krone-20; Aza-18-krone-6; Dibenzoaza-18-krone-6; Diaza-18-krone-6; Dibenzodiaza-18-krone-6; 1,4-Dithia-15-krone-5; 1,4-Dithia-18-krone-6-; 1,7-Dithiabenzo-18-krone-6; 1,10-Dithiabenzo-18-krone-6; 1,7,10,16-Tetrathia-18-krone-6.

Die Reaktion kann wie folgt durchgeführt werden: Ein Gemisch der Ausgangsstoffe II und III sowie Katalysator und Losungsmittel werden während 0,2 bis 24 h, vorzugsweise 1 bis 10 h bei der Reaktionstemperatur gehalten. Zunächst stellt man zweckmässig eine Aminophenolatlösung in einem der vorgenannten Lösungsmittel her. Hierzu wird entweder das Aminophenol zunächst in Methanol gelöst, die äquivalente Menge an Alkalimetallhydroxid zugefügt, der Alkohol durch Abziehen des Alkohols entfernt und der Rückstand in einem der vorgenannten Lösungsmittel gelöst, oder man löst das Aminophenol unter Schutzgasatmosphäre in dem Ausgangslösungsmittel, versetzt mit der äquivalenten Menge eines Alkalimetallhydroxids oder -alkoholats in fester oder gelöster Form und überführt in das Aminophenolat durch azeotrope Entwässerung mit Hilfe eines Schleppmittels oder durch Andestillieren. Zu der Lösung oder Suspension des wasserfreien Aminophenolats fügt man zweckmässig die äquivalente Menge an Halogenaromaten oder Heterohalogenaromaten in fester oder flüssiger Form oder als Lösung zu. Die Zugabe der Ausgangsstoffe kann jedoch auch so erfolgen, dass zunächst erst der Halogenaromat bzw. Halogenheteroaromat in fester oder gelöster Form vorgelegt wird und dann das Aminophenolat zugeführt wird.

Die nach dem Verfahren der Erfindung herstellbaren Verbindungen sind wertvolle Ausgangsstoffe für die Herstellung von Farbstoffen, Pharmazeutika und Pflanzenschutzmitteln. Es lassen sich aus ihnen durch Umsetzung mit Alkylisocyanaten oder mit Alkylcarbamidsäurechloriden Harnstoffe mit herbizider Wirkung herstellen (DE-OS Nr. 2411320, US-PS Nrn. 3060235, 3947437). Entsprechend liefern die erfindungsgemässen Stoffe durch Umsetzung mit Benzoylisocyanaten Benzoylharnstoffe mit insektizider Wirkung (DE-OS Nrn. 2537413, 2504982). Ferner sind sie wichtige Ausgangsstoffe für die Herstellung von bislang noch nicht publizierten substituierten Pyridazonen der Formel V

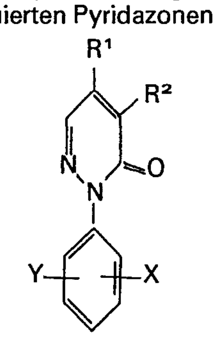 (V)

in der $R^2$ ein Halogenatom oder einen Alkoxyrest mit 1 bis 3 Kohlenstoffatomen, $R^1$ eine Amino-, Alkylamino-, Dialkylamino-, Alkoxyamino- oder Alkylalkoxyaminogruppe mit 1 bis 3 Kohlenstoffatomen je Alkyl- bzw. Alkoxyrest, wobei die Alkylreste gleich oder verschieden sein können, ein Halogenatom, einen Alkoxyrest mit 1 bis 3 Kohlenstoffatomen einen Trimethyleniminorest oder eine durch $ClH_2CC(O)$- oder $CH_3COOCH_2C(O)$-acylierte Aminogruppe, X einen substituierten Phenoxyrest der Formel

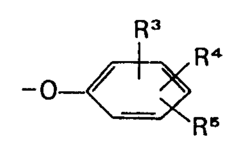

bedeutet, wobei $R^3$, $R^4$ und $R^5$ jeweils unabhängig voneinander Wasserstoff, Halogen, Nitro, Cyano, Carboxyl, Alkyl, Halogenalkyl, Alkoxy, Halogenalkoxy, Alkylmercapto, Halogenalkylmercapto, Alkylsulfinyl oder Alkylsulfonyl mit jeweils 1 bis 4 Kohlenstoffatomen bedeuten und Y Wasserstoff, Cyan, Halogen oder Nitro bedeutet, und von substituierten Harnstoffen oder der Formel VI

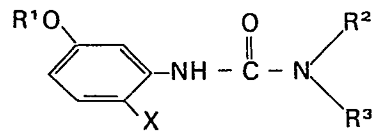 (VI)

worin $R^1$ entweder für einen substituierten Phenylrest der Formeln

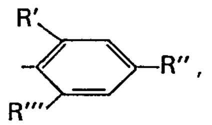

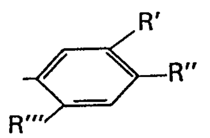 oder 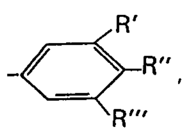

in denen R', R'' und R''' jeweils unabhängig voneinander Wasserstoff, Halogen, Nitro, Niedrigalkyl, Halogenniedrigalkyl, Cyan, Niedrigalkoxy, Halogenniedrigalkoxy, Niedrigalkylmercapto, Niedrigalkylsulfinyl oder Niedrigalkylsulfonyl bedeuten oder für einen gegebenenfalls substituierten Heteroarylrest stehen, $R^2$ Wasserstoff, Niedrigalkyl, Niedrigalkoxy, Niedrigalkenyl, Niedrigalkinyl, Cycloalkyl oder gegebenenfalls substituiertes Phenyl, $R^3$ Wasserstoff oder Niedrigalkyl bedeuten, wobei $R^2$ und $R^3$ zusammen auch für Oligomethylen oder Oxaoligomethylen stehen können, und X Wasserstoff, Halogen, Nitro, Niedrigalkyl, Halogenniedrigalkyl, Niedrigalkoxy, Halogenniedrigalkoxy oder Cyan bedeutet.

Die in den folgenden Beispielen genannten Teile bedeuten Gewichtsteile; sie verhalten sich zu Volumenteilen wie Kilogramm zu Liter.

*Beispiel 1:*

11,2 Gewichtsteile Kaliumhydroxid werden in 150 Volumenteilen absolutem Methanol gelöst, mit 21,8 Gewichtsteilen 4-Aminophenol versetzt und die klare Lösung zur Trockne im Vakuum eingeengt. Das Kaliumphenolat wird in 200 Volumenteilen Dimethylsulfoxid aufgenommen, mit 43 Gewichtsteilen 3,4-Dichlortrifluormethylbenzol und 5,2 Gewichtsteilen 18-Krone-6 versetzt und 4 h bei 140° C nachgerührt. Nach dem Abkühlen rührt man das Reaktionsgemisch in Wasser ein, dekantiert die wässerige Phase ab und nimmt den öligen Rückstand in Äther auf. Dieser wird zunächst mit verdünnter Natronlauge und Wasser behandelt, sodann mit Magnesiumsulfat getrocknet und unter Vakuum eingeengt. Der Rückstand wird einer fraktionierten Destillation unterworfen und man erhält 45 Gewichtsteile (78% der Theorie) 2'-Chlor-4'-trifluormethyl-4-aminodiphenyläther mit dem Siedepunkt 130-135° C/0,2 bar und $n_D^{25}$: 1,5593 (Fp. 58-62° C).

*Vergleich 1a:*

Versuch wie oben, jedoch ohne 18-Krone-6. Man erhält 8 Gewichtsteile 2'-Chlor-4'-trifluormethyl-4-aminodiphenyläther (14% der Theorie).

*Beispiel 2:*

Man löst 109 Gewichtsteile 3-Aminophenol in 600 Volumenteilen Dimethylsulfoxid, fügt unter Stickstoff eine konzentrierte wässerige Lösung von 68 Gewichtsteilen 86%igem Kaliumhydroxid sowie 400 Volumenteilen Toluol hinzu und kocht unter Rückfluss am Wasserabscheider, bis kein Wasser mehr abgeschieden wird. Darauf zieht man das Toluol im Vakuum ab, setzt 215 Gewichtsteile 2,4-Dichlortrifluormethylbenzol und 26 Gewichtsteile 18-Krone-6 zu und rührt 8 h bei 140° C nach. Das Reaktionsgemisch wird zunächst im Vakuum von der Hauptmenge an Lösungsmittel befreit, mit 2000 Volumenteilen Wasser versetzt und das ölige Produkt durch Abdekantieren der wässerigen Phase isoliert. Der Rückstand wird in Methylenchlorid aufgenommen, mit verdünnter Natronlauge und Wasser extrahiert, mit Magnesiumsulfat getrocknet und der Vakuumdestillation unterworfen. Man erhält 210 Gewichtsteile (73% der Theorie) 3'-Chlor-4'-trifluormethyl-3-aminodiphenyläther mit dem Siedepunkt 155 bis 160° C/0,5 bar und dem Brechungsindex $n_D^{25}$: 1,5539.

*Beispiele 3 bis 11:*

Analog den Beispielen 1 und 2 wurden die in der folgenden Tabelle genannten Verbindungen hergestellt:

*Tabelle*

| Nr. | Teile | Kaliumaminophenolat | Teile | Halogenaromat | Teile | Katalysator |
|---|---|---|---|---|---|---|
| 3 | 147 | 3-Aminophenol | 215 | 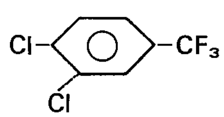 | 26 | 18-K-6 |
| 4 | 22 | 3-Aminophenol | 45 | 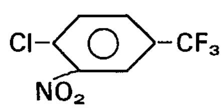 | 5,2 | 18-K-6 |

*Tabelle (Fortsetzung)*

| Nr. | Teile | Kaliumaminophenolat | Teile | Halogenaromat | Teile | Katalysator |
|---|---|---|---|---|---|---|
| 5 | 22 | 4-Aminophenol | 43 | 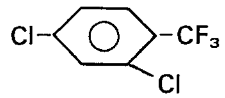 | 2,6 | 18-K-6 |
| 6 | 22 | 2-Aminophenol | 31 | 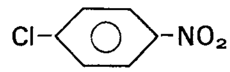 | 5,2 | 18-K-6 |
| 7 | 22 | 4-Aminophenol | 38 | 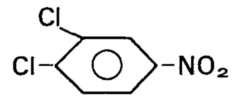 | 5,2 | 18-K-6 |
| 8 | 22 | 4-Aminophenol | 28 | 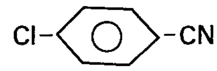 | 5,2 | Dibenzo 18-K-6 |
| 9 | 22 | 3-Aminophenol | 43 | 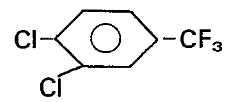 | 7,5 | Dicyclohexyl 18-K-6 |
| 10 | 22 | 3-Aminophenol | 57 | 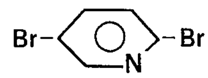 | 5,2 | 18-K-6 |
| 11 | 22 | 4-Aminophenol | 30 | 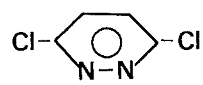 | 5,2 | 18-K-6 |
| 12 | 25 | 3-Aminothiophenol | 43 | 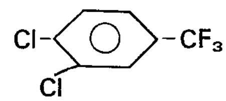 | 5,2 | 18-K-6 |

*Tabelle (Fortsetzung)*

| Nr. | Lösungsmittel | Temperatur (°C) | Zeit (h) | Teile | Endprod. Sdp. (°C/bar) | $n_D^{25}$/Fp. | Ausbeute (%) |
|---|---|---|---|---|---|---|---|
| 3 | Dimethylsulfoxid | 130 | 12 | 226 | 130-135°/0,3 | 1,5521 | 78 |
| 4 | Hexamethylen-phosphorsäuretriamid | 60 | 0,5 | 45 | 150-155°/0,2 | 1,5752 | 77 |
| 5 | Dimethylformamid | 120 | 8 | 35 | 135-140°/0,3 | 1,5578 | 61 |
| 6 | Dimethylacetamid | 120 | 0,5 | 44 | – | 119-121 °C | 96 |
| 7 | Sulfolan | 60 | 1 | 45 | | 90-93 °C | 85 |
| 8 | N-Methylpyrrolidon | 120 | 4 | 40 | | 104-106 °C | 95 |
| 9 | N-Methylpyrrolidon | 140 | 3,5 | 34 | 130-135 °C/0,3 | 1,5522 | 60 |
| 10 | Dimethylsulfoxid | 120 | 0,5 | 46 | | 73-76 °C | 87 |
| 11 | Acetonitril | 80 | 6 | 30 | | 83-87 °C | 68 |
| 12 | Dimethylsulfoxid | 140 | 3 | 50 | 160-165°/0,4 | 63-66 °C | 83 |

*Verwendungsbeispiele*

A. Herstellung von Verbindungen der Formel V

a) 28,7 Gewichtsteile 2'-Chlor-4'-trifluormethyl-3-aminodiphenyläther (= 3,2'-Chlor-4'-trifluormethylphenoxyanilin) werden mit einer Lösung von 7,6 Gewichtsteilen $NaNO_2$ in 50 Volumenteilen konz. $H_2SO_4$ und in 200 Volumenteilen Eisessig bei 10 bis 20° C nitrosiert. Die Diazoniumsalzlösung wird direkt mit 45,5 Gewichtsteilen $SnCl_2$ gelöst in 31 Volumenteilen konz. Salzsäure zum entsprechenden Hydrazin reduziert. Nach Zugabe von 16,7 Gewichtsteilen Mucochlorsäure rührt man die Reaktionslösung 10 min bei Siedetemperatur, kühlt ab und fügt 1000 Volumenteile Wasser hinzu. Der ölige Rückstand wird in $CH_2Cl_2$ gelöst, mit $MgSO_4$ getrocknet, das Lösungsmittel abgedampft und der Rückstand durch Verreiben mit Methanol kristallisiert. Man erhält 32 Gewichtsteile (74% der Theorie) 1-(N)-[3'-(2''-Chlor-4''-trifluormethylphenoxy)]-phenyl-4,5-dichlorpyridazon-6 mit dem Schmelzpunkt 89 bis 92° C.

b) 20 Gewichtsteile 1-(N)-[4'-(3''-Trifluormethylphenoxy)]phenyl-4,5-dichlorpyridazon-6

und 4 Gewichtsteile Natriummethylat werden in 80 Volumenteilen Methanol 1 h unter Rückfluss gekocht. Die Reaktionsmischung wird zur Trockne eingedampft, der Rückstand mit Wasser verrührt und abgesaugt. Nach dem Umkristallisieren aus Methanol erhält man 32 Gewichtsteile (80% der Theorie) 1-(N)-[4'-(3''-Trifluormethylphenoxy)]phenyl-4-methoxy-5-chlorpyridazon-6 vom Schmelzpunkt 113 bis 114° C.

B. Herstellung der Verbindungen der Formel VI

a) 14,4 Teile 2'-Chlor-4'-trifluormethyl-3-aminodiphenyläther werden in 50 Teilen Acetonitril gelöst und bei 20° C mit 3 Tropfen Triäthylamin und 3,2 Teilen Methylisocyanat versetzt. Man rührt 2 h bei Raumtemperatur nach, engt die Reaktionsmischung zur Trockne ein und kristallisiert den Rückstand aus Toluol um. Man erhält 10 Teile (58% der Theorie) N-Methyl-N'-3-(2'-chlor-4'-trifluormethylphenoxy)phenylharnstoff mit dem Schmelzpunkt 158 bis 161° C.

b) Zu einer Lösung von 14,4 Teilen 3,2'-Chlor-4'-trifluormethyl-3-aminodiphenyläther in 25 Teilen Pyridin fügt man bei 10° C 7,5 Teilen N,N-Dimethylcarbamidsäurechlorid portionsweise zu. Es wird ½ h auf 70 bis 75° C erwärmt, das Pyridin zum grössten Teil im Vakuum abgezogen, der Rückstand auf Wasser gegossen und mit verdünnter Salzsäure angesäuert. Der ölige Rückstand wird in Methylenchlorid aufgenommen, mit Magnesiumsulfat getrocknet und eingeengt. Nach der Chromatographie über Kieselgel mit Laufmittel Toluol/Aceton 70:3 erhält man 14 Teile (78% der Theorie) N,N-Dimethyl-N'-3-(2'-chlor-4'-trifluormethylphenoxy)phenylharnstoff mit dem Schmelzpunkt 96 bis 98° C.

Der Einfluss von Vertretern der neuen substituierten Pyridazone der Formel V auf das Wachstum von erwünschten und unerwünschten Pflanzen wird anhand von Gewächshausversuchen vorgeführt:

Als Kulturgefässe dienten Plastikblumentöpfe mit 300 $cm^3$ Inhalt und lehmiger Sand mit etwa 1,5% Humus als Substrat. Die Samen der Testpflanzen wurden nach Arten getrennt flach eingesät. Unmittelbar danach erfolgte bei Vorauflaufbehandlung das Aufbringen der Wirkstoffe auf die Erdoberfläche. Sie wurden hierbei in Wasser als Verteilungsmittel suspendiert oder emulgiert und mittels fein verteilender Düsen gespritzt. Bei dieser Applikationsmethode handelt es sich um eine Aufwandmenge entsprechend 3,0 kg Wirkstoff/ha. Nach dem Aufbringen der Mittel wurden die Gefässe leicht beregnet, um Keimung und Wachstum in Gang zu bringen. Danach deckte man die Gefässe mit durchsichtigen Plastikhauben ab, bis die Pflanzen angewachsen waren. Diese Abdeckung bewirkte ein gleichmässiges Keimen der Testpflanzen, sofern dies nicht durch die Wirkstoffe beeinträchtigt wurde. Zum Zwecke der Nachauflaufbehandlung zog man die Testpflanzen je nach Wuchsform erst bis zu einer Wuchshöhe von 3 bis 10 cm an und behandelte sie danach. Zur Nachauflaufbehandlung wurden entweder direkt gesäte und in den gleichen Gefässen aufgewachsene Pflanzen ausgewählt, oder sie wurden erst als Keimpflanzen getrennt angezogen und einige Tage vor der Behandlung in die Versuchsgefässe verpflanzt. Die Aufwandmengen für die Nachauflaufbehandlung waren 0,25 kg/ha und 0,5 kg/ha Wirkstoff. Als Vergleichsmittel wurden die bekannten Wirkstoffe 1-Phenyl-4,5-dimethoxypyridazon-(6) (A) und 1-m-Trifluormethylphenyl-4,5-dimethoxypyridazon-(6) (B) in den gleichen Aufwandmengen gewählt. Eine Abdeckung unterblieb bei der Nachauflaufbehandlung. Die Aufstellung der Versuche erfolgte im Gewächshaus, wobei für wärmeliebende Arten wärmere Bereiche (20 bis 30° C) und für solche gemässigter Klimate 15 bis 25° C bevorzugt wurden. Die Versuchsperiode erstreckte sich über 2 bis 4 Wochen. Während dieser Zeit wurden die Pflanzen gepflegt und ihre Reaktion auf die einzelnen Behandlungen ausgewertet. Bewertet wurde nach einer Skala von 0 bis 100.

Dabei bedeutet 0 keine Schädigung oder normaler Auflauf und 100 kein Aufgang der Pflanzen bzw. völlige Zerstörung zumindest der oberirdischen Teile.

Die Ergebnisse zeigen, dass die neuen Verbindungen bei der vorzugsweise durchgeführten Nachauflaufbehandlung insgesamt eine ähnliche, gegen einige unerwünschte Arten sogar eine bessere herbizide Aktivität haben als der bekannte Vergleichswirkstoff. Besonders hervorzuheben ist jedoch die wesentlich günstigere Verträglichkeit der neuen Verbindungen für Kulturpflanzen, vorzugsweise aus der Familie der Gramineen. Bei Vorauflaufanwendung wird ebenfalls eine herbizide Wirkung beobachtet.

In Betracht kommen beispielsweise die folgenden Kulturen:

| Botanischer Name | Deutscher Name | Englischer Name |
|---|---|---|
| *Allium cepa* | Küchenzwiebel | onions |
| *Ananas comosus* | Ananas | pineapple |
| *Arachis hypogaea* | Erdnuss | peanuts (groundnuts) |
| *Asparagus officinalis* | Spargel | asparagus |
| *Aventa sativa* | Hafer | oats |
| *Beta vulgaris spp. altissima* | Zuckerrübe | sugarbeets |
| *Beta vulgaris spp. rapa* | Futterrübe | fooder beets |
| *Beta vulgaris spp. esculenta* | Rote Rübe | table beets, red beets |
| *Brassica napus var. napus* | Raps | rape seed |
| *Brassica napus var. naprobrassica* | Kohlrübe | |

| Botanischer Name | Deutscher Name | Englischer Name |
|---|---|---|
| *Brassica napus var. rapa* | Weisse Rübe | turnips |
| *Brassica rapa var. silvestris* | Rüben | |
| *Camellia sinensis* | Teestrauch | tea plants |
| *Carthamus tinctorius* | Saflor-Färbedistel | safflower |
| *Carya illinoinensis* | Pekannussbaum | pecan trees |
| *Citrus limon* | Zitrone | lemon |
| *Citrus maxima* | Pampelmuse | grapefruits |
| *Citrus reticulata* | Mandarine | |
| *Citrus sinensis* | Apfelsine, Orange | orange trees |
| *Coffea arabica (Coffea cane phora, Coffea liberica)* | Kaffee | coffee plants |
| *Cucumis melo* | Melone | melons |
| *Cucumis sativus* | Gurke | cucumber |
| *Cynodon dactylon* | Bermudagras | Bermudagrass in turfs and lawns |
| *Daucus carota* | Möhre | carrots |
| *Elaeis guineensis* | Ölpalme | oil palms |
| *Fragaria vesca* | Erdbeere | strawberries |
| *Glycine max* | Sojabohne | soybeans |
| *Gossypium hirsutum (Gossypium arboreum Gossypium herbaceum Gossypium vitifolium)* | Baumwolle | cotton |
| *Helianthus annuus* | Sonnenblume | sunflowers |
| *Helianthus tuberosus* | Topinambur | |
| *Hevea brasiliensis* | Parakautschukbaum | rubber plants |
| *Hordeum vulgare* | Gerste | barley |
| *Humulus lupulus* | Hopfen | hop |
| *Ipomoea batatas* | Süsskartoffeln | sweet potato |
| *Juglans regia* | Walnussbaum | walnut trees |
| *Lactuca sativa* | Kopfsalat | lettuce |
| *Lens culinaris* | Linse | lentils |
| *Linum usitatissimum* | Faserlein | flax |
| *Lycopersiocon lycopersicum* | Tomate | tomato |
| *Malus spp.* | Apfel | apple trees |
| *Manihot esculenta* | Maniok | cassava |
| *Medicago sativa* | Luzerne | alfalfa (lucerne) |
| *Mentha piperita* | Pfefferminze | peppermint |
| *Musa spp.* | Obst- u. Mehlbanane | banana plants |
| *Nicotiana tabacum (N. rustica)* | Tabak | tobacco |
| *Olea europaea* | Ölbaum | olive trees |
| *Oryza sativa* | Reis | rice |
| *Panicum miliaceum* | Rispenhirse | |
| *Phaseolus lunatus* | Mondbohne | limabeans |
| *Phaseolus mungo* | Erdbohne | mungbeans |
| *Phaseolus vulgaris* | Buschbohnen | snapbeans, green beans, dry beans |
| *Pennisetum glaucum* | Perl- oder Rohrkolbenhirse | |
| *Petroselinim crispum spp. tuberosum* | Wurzelpetersilie | parsley |
| *Picea abies* | Rotfichte | Norway spruce |
| *Abies alba* | Weisstanne | fire |
| *Pinus spp.* | Kiefer | pine trees |
| *Pisum sativum* | Gartenerbse | English peas |
| *Prunus avium* | Süsskirsche | cherry trees |
| *Prunus domestica* | Pflaume | plum trees |
| *Prunus dulcis* | Mandelbaum | almond trees |
| *Prunus persica* | Pfirsich | peach trees |
| *Prunus communis* | Birne | pear trees |

| Botanischer Name | Deutscher Name | Englischer Name |
|---|---|---|
| *Ribes sylvestre* | Rote Johannisbeere | red currants |
| *Ribes uva-crispa* | Stachelbeere | |
| *Ricinus communis* | Rizinus | |
| *Saccharum officinarum* | Zuckerrohr | sugar cane |
| *Secale cereale* | Roggen | rye |
| *Sesamum indicum* | Sesam | sesame |
| *Solanum tuberosum* | Kartoffel | Irish potato |
| *Sorghum bicolor (s. vulgare)* | Mohrenhirse | sorghum |
| *Sorghum dochna* | Zuckerhirse | |
| *Spinacia oleracea* | Spinat | spinach |
| *Theobroma cacao* | Kakaobaum | cacao plants |
| *Trifolium pratense* | Rotklee | red clover |
| *Triticum aestivum* | Weizen | wheat |
| *Vaccinium carymbosum* | Kulturheidelbeere | blueberry |
| *Vaccinium vitis-idaea* | Preisselbeere | cranberry |
| *Vicia faba* | Pferdebohnen | tick beans |
| *Vigna sinensis (V. unguiculata)* | Kuhbohne | cow peas |
| *Vitis vinifera* | Weinrebe | grapes |
| *Zea mays* | Mais | Indian corn, sweet corn, maize |

Die Verbindungen der Formel VI eignen sich beispielsweise zur Nachauflaufbehandlung gemäss der geschilderten Methode von *Glycine max.* (Sojabohnen), *Grossypium hirsutum* (Baumwolle), *Hordeum vulgare* (Gerste), *Sorghum bicolor* (Möhren, Kulturhirse), *Triticum aestivum* (Weizen) und *Zea mays* (Mais) gegen folgende unerwünschte Pflanzen: *Amaranthus retroflexus* (Zurückgekrümmter Fuchsschwanz), *Cassia spp.* (Cassiaarten), *Centaurea cyanus* (Kornblume), *Chenopodium album* (Weisser Gänsefuss), *Chrysanthemum segetum* (Saatwucherblume), *Echinochloa crus galli* (Hühnerhirse), *Euphorbia geniculata* (Südamerik. Wolfsmilchart), *Lamium spp.* (Taubnesselarten), *Matricaria spp.* (Kamillearten), *Sesbania exaltata* (Turibaum), *Sinapis alba* (Weisser Senf), *Solanum nigrum* (Schwarzer Nachtschatten), *Chenopodium spp.* (Gänsefussart).

**Patentansprüche**

1. Verfahren zur Herstellung von substituierten Anilinen der Formel

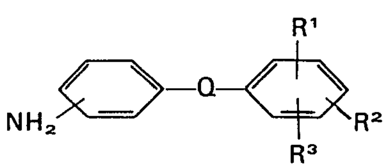

(I)

worin in dem aminogruppenfreien aromatischen Ring 1 oder 2 CH-Gruppen durch Stickstoffatome ersetzt sein können, die Reste $R^1$, $R^2$ und $R^3$ gleich oder verschieden sein können und Wasserstoffatome, Halogenatome, Nitrogruppen, Cyangruppen, Trihalogenmethylgruppen, $C_{1\ \mathrm{bis}\ 4}$-Alkoxyreste, $C_{1\ \mathrm{bis}\ 4}$-Alkylmercaptoreste, aliphatische Reste mit 1 bis 4 Kohlenstoffatomen, aliphatische Sulfonylreste mit 1 bis 4 Kohlenstoffatomen, aliphatische Sulfoxidreste mit 1 bis 4 Kohlenstoffatomen oder Carbalkoxyreste mit 2 bis 5 Kohlenstoffatomen und Q ein Sauerstoff- oder Schwefelatom bezeichnen, durch Umsetzung eines Phenolsalzes mit aromatischen oder heterocyclischen Halogenverbindungen, dadurch gekennzeichnet, dass man Verbindungen der Formel

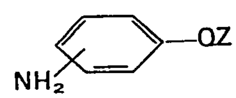

(II)

worin Q die vorgenannte Bedeutung besitzt und Z ein Metallkation bedeutet, mit Halogenaromaten oder Halogenheterocyclen der Formel

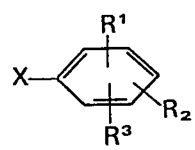

(III)

worin X ein Halogenatom bedeutet, $R^1$, $R^2$ und $R^3$ die vorgenannten Bedeutungen besitzen und 1 oder 2 CH-Gruppen im Ring durch Stickstoffatome ersetzt sein können, in Gegenwart eines Kronenäthers als Katalysator und polarer aprotischer Lösungsmittel, bei einer Temperatur zwischen 80 und 280° C umsetzt.

2. 2'-Chlor-4'-trifluormethyl-3-aminodiphenyläther.
3. 3'-Chlor-4'-trifluormethyl-3-aminodiphenyläther.
4. 2'-Nitro-4'-trifluormethyl-3-aminodiphenyläther.
5. 3, 2'-Brom-5'-pyridinoxyanilin,
6. 2'-Chlor-4'-trifluormethyl-3-aminodiphenylthioäther.

## Claims

1. A process for the preparation of a substituted aniline of the formula

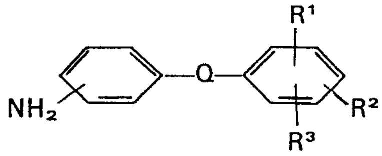

(I)

where 1 or 2 CH groups in the aromatic ring which is free from amino groups can be replaced by nitrogen atoms, $R^1$, $R^2$ and $R^3$ may be identical or different and each is hydrogen, halogen, nitro, cyano, trihalomethyl, $C_{1-4}$-alkoxy, $C_{1-4}$-alkylmercapto, an aliphatic radical of 1 to 4 carbon atoms, an aliphatic sulfonyl radical of 1 to 4 carbon atoms, an aliphatic sulfoxide radical of 1 to 4 carbon atoms or a carbalkoxy radical of 2 to 5 carbon atoms, and Q is oxygen or sulfur, by reacting a phenolic salt with an aromatic or heterocyclic halogen compound, wherein a compound of the formula

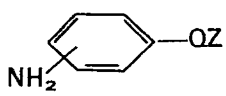

(II)

where Q has the above meanings and Z is a metal cation, is reacted with a haloaromatic or haloheterocyclic compound of the formula

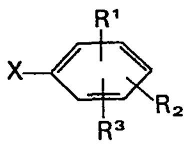

(III)

where X is halogen, $R^1$, $R^2$ and $R^3$ have the above meanings and 1 or 2 CH groups in the ring can be replaced by nitrogen atoms, in the presence of a crown ether as catalyst and of a polar aprotic solvent, at from 80 to 280° C.

2. 2'-Chloro-4'-trifluoromethyl-3-aminodiphenylether.
3. 3'-Chloro-4'-trifluoromethyl-3-aminodiphenylether.
4. 2'-Nitro-4'-trifluoromethyl-3-aminodiphenylether.
5. 3, 2'-Bromo-5'-pyridinoxyaniline.
6. 2'-Chloro-4'-trifluoromethyl-3-aminodiphenylthioether.

## Revendications

1. Procédé pour la préparation d'anilines substituées de formule

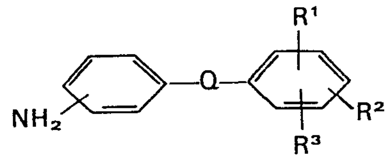

(I)

dans laquelle un ou deux groupes CH peuvent être remplacés par des atomes d'azote dans le noyau aromatique dépourvu de groupe amine, les radicaux $R^1$, $R^2$ et $R^3$ peuvent être semblables ou différents et représentent des atomes d'hydrogène, des atomes d'halogènes, des groupes nitro, des groupes cyano, des gıoupes trihalogénométhyle, des radicaux alcoxy en $C_{1-4}$, des radicaux alcoyl-(en $C_{1-4}$)-mercapto, des radicaux aliphatiques à 1-4 atomes de carbone, des radicaux sulfonyle aliphatiques à 1-4 atomes de carbone, des radicaux sulfoxyde aliphatiques à 1-4 atomes de carbone ou des radicaux carbalcoxy à 2-5 atomes de carbone, et Q représente un atome d'oxygène ou de soufre, par réaction d'un sel de phénol avec des composés aromatiques ou hétérocycliques halogénés, caractérisé en ce qu'on fait réagir, en présence d'un éther en couronne servant de catalyseur et d'un solvant aprotique polaire, à une température comprise entre 80 et 280° C, des composés de formule

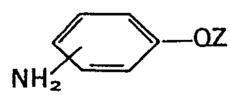

(II)

dans laquelle Q a la signification donnée précédemment, et Z représente un cation métallique, avec des carbures aromatiques halogénés ou des carbures hétérocycliques halogénés de formule

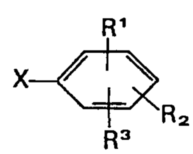

(III)

dans laquelle X représente un atome d'halogène, $R^1$, $R^2$ et $R^3$ ont les significations données précédemment et un ou deux groupes dans le noyau peuvent être remplacés par des atomes d'azote.

2. 2'-Chloro-4'-trifluorométhyl-3-aminodiphényléther.
3. 3'-Chloro-4'-trifluorométhyl-3-aminodipéhnyléther.
4. 2'-Nitro-4'-trifluorométhyl-3-aminodiphényléther.
5. 3, 2'-Bromo-5'-pyridinoxyaniline.
6. 2'-Chloro-4'-trifluorométhyl-3-aminodiphénylthioéther.